# EUROPEAN PATENT SPECIFICATION

(11) **EP 2 972 364 B1**
(45) Date of publication and mention of the grant of the patent: **09.05.2018**
(21) Application number: 14779170.1
(22) Date of filing: 12.03.2014
(51) Int. Cl.: G01N 33/561, G01N 33/49, G01N 33/53, G01N 33/543, G01N 33/68, G01N 27/447

(54) **MICROFLUIDIC DEVICE FOR IMMUNOBLOTTING**
MIKROFLUIDISCHE VORRICHTUNG FÜR IMMUNOBLOTTING
DISPOSITIF MICROFLUIDIQUE POUR L'IMMUNOTRANSFERT

(30) Priority: 12.03.2013 US 201361777682 P
(43) Date of publication of application: 20.01.2016
(73) Proprietor: The Regents of The University of Michigan, Ann Arbor, Michigan 48109 (US)
(72) Inventor: GOONEWARDENA, Sascha, N., Ann Arbor, MI 48109 (US); GASPER, Christopher, Ann Arbor, MI 48109 (US); BAKER, Jr., James, R., Ann Arbor, MI 48103 (US); LEROUEIL, Pascale, R., Ann Arbor, MI 48109 (US); CHANG, Huai, Ning, Ann Arbor, MI 48106 (US)
(74) Representative: Glawe, Delfs, Moll
(86) International application number: PCT/US2014/024684
(87) International publication number: WO 2014/165185

(56) References cited:
- WO-A1-2007/060580
- WO-A2-2011/106693
- US-A1- 2011 177 618
- US-A1- 2011 312 804
- US-A1- 2012 202 219
- US-A1- 2012 211 373
- GWENDOLYN J. ANDERSON ET AL: "Western Blotting Using Capillary Electrophoresis", ANALYTICAL CHEMISTRY (AMERICAN CHEMICAL SOCIETY), vol. 83, no. 4, 15 February 2011 (2011-02-15), pages 1350-1355, XP055311405, Washington DC, USA ISSN: 0003-2700, DOI: 10.1021/ac102671n
- H. N. CHANG ET AL.: "Profiling Inflammatory Responses with Microfluidic Immunoblotting", PLOS ONE (PUBLIC LIBRARY OF SCIENCE), vol. 8, no. 11, 27 November 2013 (2013-11-27), pages 1-11, XP055311059, San Francisco CA USA DOI: 10.1371/journal.pone.0081889

## Description

### FIELD

The present invention provides microfluidic devices, systems, and methods for use in immunoblotting applications. In particular, devices and methods provided herein have the advantages of traditional Western blotting with increased throughput and multiplexability, and decreased time, sample, and reagent requirements.

### BACKGROUND

Traditional Western blot analysis (or immunoblotting) is used to detect a specific protein in a cell, tissue, organ, body fluid, etc. The technique is a mainstay of biochemistry and molecular biology. Proteins in a sample are separated by gel electrophoresis (e.g., SDS-PAGE), and then transferred from the gel to a membrane (e.g., nitrocellulose or PVDF). The membrane is then stained with antibodies specific for a target protein. Although reliably yielding useful information, the technique is: time consuming, requiring of technical skill, reagent intensive, limited throughput, and poorly suited for multiplexing. What is needed is a technique that provides the advantages of traditional Western blotting with the increased throughput, multiplexability, and reduced reagent usage.

WO 2011/106693 A2 discloses systems and methods for integrating the electrophoresis and blotting of samples. The disclosed technology relates to electrophoresis and blotting systems including capillaries or microfluidic channels.

US 2011/312804 A1 relates to a microfluidic device having a channel having an inlet, an outlet and an aperture between the inlet and the outlet, wherein, the aperture has a geometry that does not allow a meniscus of the fluid flow to anchor to the aperture, such that fluid flow from the inlet towards the outlet is uninterrupted.

US 2011/177618 A1 discloses multi-directional microfluidic devices and methods for using the same. The microfluidic devices are configured to subject a sample to two or more directionally distinct flow fields, and include a separation medium and a binding medium, where the binding medium is in fluid communication with the separation medium.

US 2012/211373 A1 relates to devices, systems and methods for measuring cell barrier function, in particular to microfluidic devices for use in *in vitro* models of the blood-brain barrier and modeling the transport across this barrier.

US 2012/202219 A1 discloses novel synthetic prostate specific antigen (PSA)-targeted capture agents that specifically bind PSA.

WO 2007/060580 A1 relates to a microfluidic device for detection of a substance in a sample fluid, and to a cartridge for performing a biological assay, containing such a device.

Anderson et al., Western blotting using capillary electrophoresis, Analytical Chemistry, 83(4), 2011, pages 1350- 1355, describes microscale Western blotting system based on separating the sodium-dodecyl sulfate protein complexes by capillary gel electrophoresis followed by deposition onto a blotting membrane for immunoassay.

Chang et al., Profiling inflammatory responses with microfluidic immunoblotting, PLOS ONE, 8(11), 2013, pages 1-11, describes profiling inflammatory responses with microfluidic immunoblotting.

### SUMMARY OF THE INVENTION

The present invention provides microfluidic devices, systems, and methods for use in immunoblotting applications. In particular, devices and methods provided herein have the advantages of traditional Western blotting with increased throughput and multiplexability, and decreased time, sample, and reagent requirements.

In some embodiments, the present invention provides microfluidic immunoblotting devices comprising a flat membrane-contacting surface and a plurality of non-connected parallel microfluidic channels, wherein the length of said microfluidic channels are open to the membrane-contacting surface. In some embodiments, the microfluidic channels are spatially grouped into two or more channel sets. In some embodiments, each of the two or more channel sets comprises 2-20 microfluidic channels. In some embodiments, the distance separating microfluidic channels with a channel set is less than the distance between channel sets. In some embodiments, a microfluidic channel (e.g., each of the microfluidic channels) comprises a reservoir at one end of the microfluidic channel. In some embodiments, a microfluidic channel (e.g., each of the microfluidic channels) comprises reservoirs at both ends of the microfluidic channel. In some embodiments, each of the microfluidic channels is 50-300 µm wide. In some embodiments, each of the microfluidic channels is 100-200 µm wide. In some embodiments, each of the microfluidic channels is 140-160 µm wide. In some embodiments, each of the microfluidic channels is 30-200 µm deep. In some embodiments, each of the microfluidic channels is 50-150 µm deep. In some embodiments, each of the microfluidic channels is 90-110 µm deep. In some embodiments, the ratio of width to depth of the microfluidic channels is less than 10:1 (e.g., <9:1, <8:1, <7:1, <6:1, <5:1, <4:1, <3:1, <2:1). In some embodiments, the ratio of width to depth of the microfluidic channels is between 3:1 and 1:3 (e.g., 2:1 to 1:2). In some embodiments, the ratio of width to depth of the microfluidic channels is 9:1, 8:1, 7:1, 6:1,5:1,4:1,3:1,2:1, 1.5:1, 1:1, 1:1.5, 1:2, 1:3, 1:4. 1:5, 1:6, 1:7, 1:8, or 1:9. In some embodiments, the ratio of width to depth of the microfluidic channels is between 3:1 and 1:3 (e.g., 2:1 to 1:2). In some embodiments, the device comprises polydimethylsiloxane (PDMS). In some embodiments, the device consists essentially of polydimethylsiloxane (PDMS). In some embodiments, the present invention provides methods of immunoblotting comprising applying antibody solutions to a membrane using a device of the present invention.

In some embodiments, the present invention provides systems (e.g., assays) comprising a device of the present invention and a membrane. In some embodiments, an activating solution is located or placed between said device and said membrane. In some embodiments, the activating solution comprises polysorbate-20 (Tween-20) and BSA. In some embodiments, the activating solution comprises 0.01-0.5% Tween-20 and 0.01-0.5% BSA. In some embodiments, the activating solution comprises 0.05-0.15% Tween-20 and 0.05-0.15% BSA. In some embodiments, the activating solution comprises 0.1% Tween-20 and 0.1% BSA. In some embodiments, the membrane comprises membrane nitrocellulose or polyvinylidene fluoride (PVDF). In some embodiments, the membrane comprises peptides, polypeptides, or proteins on or within it. In some embodiments, systems further comprise one or more antibody-containing solutions within the microfluidic channels. In some embodiments, each microfluidic channel of a channel set comprises a different antibody-containing solution. In some embodiments, each channel set comprises the same combination of antibody-containing solutions. In some embodiments, each microfluidic channel comprises a different antibody-containing solution. In some embodiments, the present invention provides methods of immunoblotting comprising applying antibody solutions to a membrane using system of the present invention.

### BREIF DESCRIPTION OF THE DRAWINGS

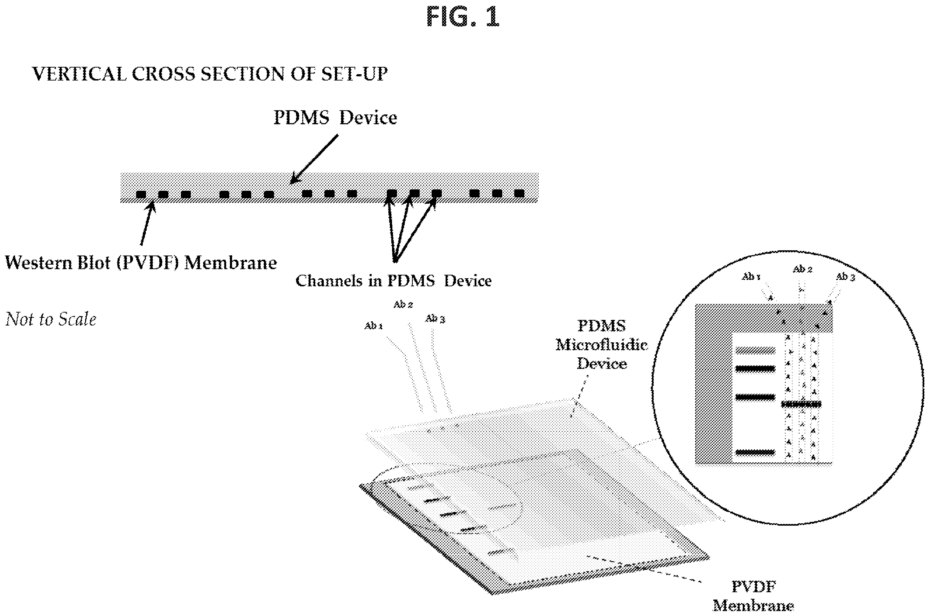

Figure 1 shows a schematic of a microfluidic immunoblotting device and an interface with a membrane. Three microfluidic channels overlie each sample lane that can be used to probe for three different proteins per lane.

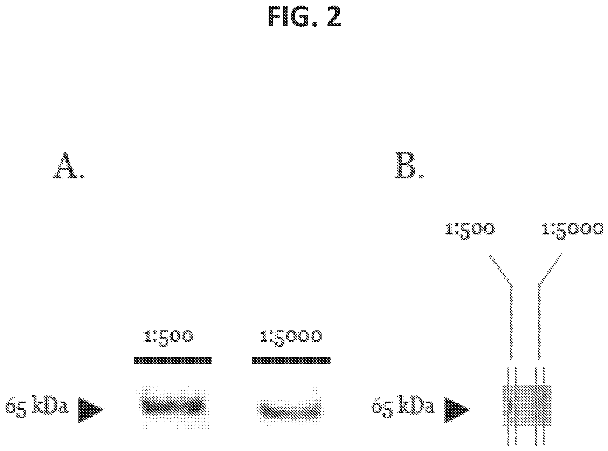

Figure 2 shows antibody dilutions in clinical PBMC samples. (A) Traditional immunoblotting with primary antibody against RelA/p65 using 1:500 and 1:5000 antibody dilutions. (B) Microfluidic immunoblot probing for RelA/p65 using the same conditions as demonstrating similar signal intensities and the flexibility to do several dilutions on the same sample.

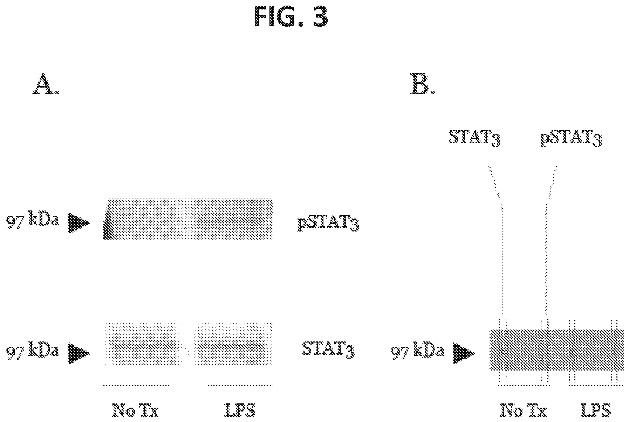

Figure 3 shows detection of STAT3 phosphorylation in response to inflammatory stimuli. (A) Traditional immunoblots of RAW264.7 cell lysates showing phosphorylation of STAT3 in response to LPS stimulation. (B) Microfluidic immunoblot on same RAW264.7 cell lysates as (A). The microfluidic device allows for simultaneous monitoring of phospho-STAT3 and STAT3 in the same sample without the need for stripping or reprobing of the PVDF membrane.

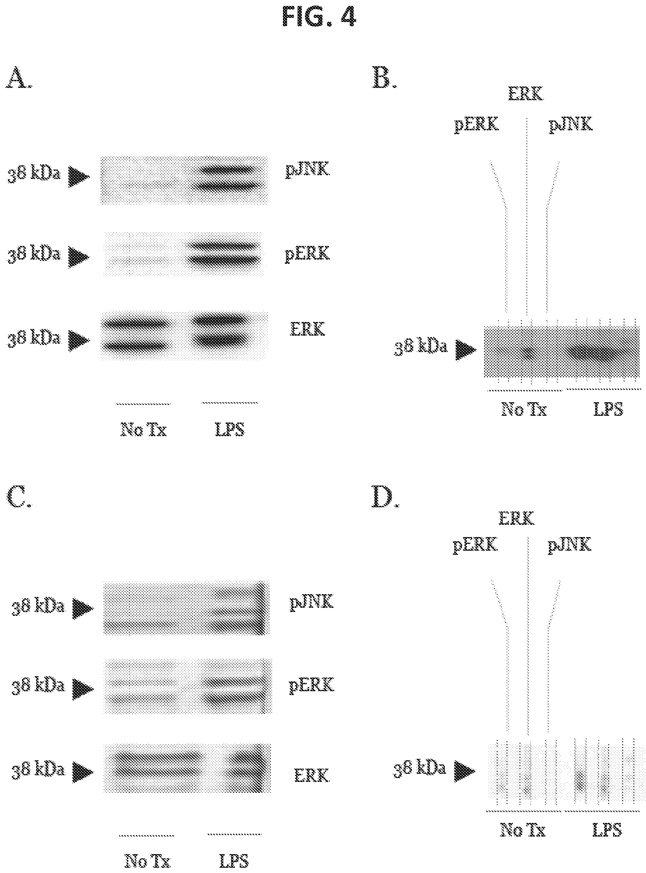

Figure 4 shows protein immunoblots of the MAPK pathway with two different chemiluminescent detection modalities. (A) Traditional immunoblots of RAW264.7 cell lysates with or without LPS for 45 minutes. Membranes were probed for phospho-JNK and phosphor-ERK using total ERK as the loading control. Primary antibodies were detected using HRP chemiluminescent techniques. (B) Microfluidic immunoblots mirroring conditions in (A). (C) and (D) are identical to (A) and (B) except they were performed using alkaline phosphatase chemiluminescent secondary antibodies to detect primary antibodies.

### DEFINITIONS

The term "antibody" herein is used in the broadest sense and specifically covers monoclonal antibodies, polyclonal antibodies, dimers, multimers, multispecific antibodies (e.g., bispecific antibodies), and antibody fragments, so long as they exhibit the desired biological activity (Miller et al (2003) Jour. of Immunology 170:4854-4861). Antibodies may be murine, human, humanized, chimeric, or derived from other species. An antibody is a protein generated by the immune system that is capable of recognizing and binding to a specific antigen. (Janeway, C., Travers, P., Walport, M., Shlomchik (2001) Immuno Biology, 5th Ed., Garland Publishing, New York). A target antigen generally has numerous binding sites, also called epitopes, recognized by CDRs on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, i.e., a molecule that contains an antigen binding site that immunospecifically binds an antigen of a target of interest or part thereof, such targets including but not limited to, cancer cell or cells that produce autoimmune antibodies associated with an autoimmune disease. The immunoglobulin can be of any type (e.g., IgG, IgE, IgM, IgD, and IgA), class (e.g., IgG1, IgG2, IgG3, IgG4, IgA1 and IgA2) or subclass of immunoglobulin molecule. The immunoglobulins can be derived from any species, including human, murine, or rabbit origin.

As used herein, the term "antibody fragment" refers to a portion of a full length antibody, generally the antigen binding or variable region thereof. Examples of antibody fragments include Fab, Fab', F(ab').sub.2, and Fv fragments; diabodies; linear antibodies; fragments produced by a Fab expression library, anti-idiotypic (anti-Id) antibodies, CDR (complementary determining region), and epitope-binding fragments of any of the above which immunospecifically bind to antigens.

As used herein, the phrase "multiplex" or grammatical equivalents refers to the detection, analysis or amplification of more than one target sequence of interest. In one embodiment multiplex refers to >3, >5, >8, >10, >20> 50, >100, etc. "Multiplexability" refers to the quality of a particular device, reagent, system, platform, kit, etc. to be used in a multiplex fashion.

As used herein, the term "sample" is used in its broadest sense. In one sense, it is meant to include cells (e.g., human, bacterial, yeast, and fungi), an organism, a specimen or culture obtained from any source, as well as biological and environmental samples. Biological samples may be obtained from animals (including humans) and refers to a biological material or compositions found therein, including, but not limited to, bone marrow, blood, serum, platelet, plasma, interstitial fluid, urine, cerebrospinal fluid, nucleic acid, DNA, tissue, and purified or filtered forms thereof. Environmental samples include environmental material such as surface matter, soil, water, crystals and industrial samples. Such examples are not however to be construed as limiting the sample types applicable to the present invention.

### DETAILED DESCRIPTION OF EMBODIMENTS OF THE INVENTION

The present invention provides microfluidic devices, systems, and methods for use in immunoblotting applications. In particular, devices and methods provided herein have the advantages of traditional Western blotting with increased throughput and multiplexability, and decreased time, sample, and reagent requirements.

Since its inception in 1979, protein immunoblotting has become the standard technique for profiling proteins in molecular biology and clinical diagnostics (Towbin et al. 1979) Although, traditional protein immunoblotting remains a powerful technique, it has limitations including its slow throughput, the requirement for relatively large sample and antibody amounts, and the inability to probe for multiple proteins simultaneously (Wu et al. 2007).

Several variations on protein immunoblotting have evolved to overcome some of the early limitations, including membrane stripping and reprobing that allows for sequential detection of distinct proteins, and the introduction of fluorescent secondary antibodies, that can be used to increase the different proteins detected from samples. Despite the improvements, these variations have their own limitations, including loss of signal and signal variability. Additionally, none of these variations address the large sample and antibody amounts required by traditional immunoblotting.

Microfluidic technology has been applied to molecular biology and to clinical diagnostics. The small volumes and precise spatial control afforded by microfluidics make it an exciting complement to existing molecular biology technologies. The Herr group incorporated microfluidic technology with traditional protein immunoblotting (He and Herr 2009; Hughes and Herr 2012). While an improvement, this approach could only detect single proteins and did not have the flexibility to interface with existing immunoblotting platforms. More recently, a microfluidic device that integrates the separation, transfer, and detection components of immunoblotting into a single device has been developed (Tia et al. 2011). While the speed and multiplexing ability of this device is a significant enhancement, the ability to probe for specific proteins in complex biological matrices has yet to be demonstrated with such integrated microfluidic devices. Pan and colleagues made further advancements by introducing a fluorescence based microfluidic device that could detect multiple proteins and is compatible with existing protein immunoblotting methods (Pan et al. 2010). None of these microfluidic devices allow for simultaneously probing for multiple proteins, and interface with existing immunoblotting equipment and chemiluminescent detection methods.

While traditional Western blotting can yield important information (e.g., about the interaction between a biological system (e.g., humans) and an outside stimulus (e.g., an anti-inflammatory drug)), they are expensive, both in terms of reagents and time. In certain embodiments, the present invention provides devices and methods that provide similar and/or greater information content as traditional Western blotting, but with decreased reagent and time consumption, and increased throughput and multiplexability. In some embodiments, the present invention provides a microfluidic device comprising sets of microfluidic channels configured to overlay the lanes present on a protein-containing membrane. In various embodiments, proteins on the membrane are probed by antibodies within the microfluidic channels. In some embodiments, a single protein lane on a membranes is overlayed by two or more microfluidic channels (e.g., 2, 3, 4, 5, 6, 7, 8, 9, 10, or more), each containing a different antibody for probing the protein lane.

In some embodiments, the present invention provides numerous advantages over traditional immunoblotting. For example, instead of incubating the entire membrane with a single type of primary antibody, a much smaller quantity of antibody is provided in a single microfluidic channel per protein lane. In embodiments in which multiple microfluidic channels are provided per protein lane, a single protein lane may be probed by multiple different antibodies simultaneously. In some embodiments, the present invention allows for probing with more types of antibodies (e.g., 2... 5... 10... 20, or more per lane of a gel) using less reagent (e.g., microfluidic channel vs. incubating the entire membrane), less sample (e.g., a single lane on a gel vs. a different gel for each different antibody), and less time (e.g., a single gel and membrane transfer vs. a different gel and transfer) than traditional immunoblotting. In some embodiments, from a time perspective, devices and method of the present invention can complete 3, 5, 10, 20, or more immunoblots in the time it takes to complete one traditional Western blot. In some embodiments, from a cost perspective, devices and methods of the present invention can complete an immunoblot for less than 1% of the cost of a traditional Western Blot.

### A. Device

In certain embodiments, microfluidic devices are provided that comprises one or more sets (e.g., 1, 2, 3, 4, 5, 6, 7, 8, 9, 10, 11, 12, 13, 14, 15, 16, 17, 18, 19, 20, or more) of one or more microfluidic channels (e.g., 1, 2, 3, 4, 5, 6, 7, 8, 9, 10, 11, 12, 13, 14, 15, 16, 17, 18, 19, 20, or more). In some embodiments, the channels run along the flat surface of the device, such that fluid contained within the channels is exposed to the surface of the device. In some embodiments, each channel comprises a reservoir or other fluid-introduction means for introduction of antibody solution into the microfluidic channel.

In some embodiments, a microfluidic device does not require valves, pumping, mixing chambers, and/or other more complex elements that are common to microfluidic devices, but increase the cost of a device and the complexity of its use. In some embodiments, a microfluidic device does not require integration with gel electrophoresis in order to carry out immunoblotting.

In some embodiments, a microfluidic device comprises one or more sets of channels, each containing one or more channels. Each set is configured to probe a single lane on of a protein gel. In some embodiments, each channel of a set is configured to probe the protein lane with a different antibody. Each channel set may comprise the same or different number of channels.

In some embodiments, the device may be of any suitable size and dimensions. In some embodiments, a device is configured to match the dimensions of a particular gel or membrane. In some embodiments, a device is configured to be usable with gels and membranes of different sizes and/or dimensions. In some embodiments, devices comprise a footprint (e.g., membrane-contacting face) with dimensions between 1 cm and 50 cm or more (e.g., 1cm x 1 cm, 4 cm x 6 cm, 8 cm x 6 cm, 5 cm x 20 cm, 20 cm x 40 cm, etc.). In some embodiments, the length and/or width of the membrane-contacting face of a microfluidic device is 1 cm... 2 cm... 3 cm... 4 cm... 5 cm... 6 cm... 7 cm... 8 cm... 9 cm... 10 cm... 11 cm... 12 cm... 13 cm... 14 cm... 15 cm...16 cm... 17 cm... 18 cm... 19 cm... 20 cm... 21 cm... 22 cm... 23 cm... 24 cm... 25 cm... 26 cm... 27 cm... 28 cm... 29 cm... 30 cm... 31 cm... 32 cm... 33 cm... 34 cm... 35 cm... 36 cm... 37 cm... 38 cm... 39 cm... 40 cm... 41 cm... 42 cm... 43 cm... 44 cm... 45 cm... 46 cm... 47 cm... 48 cm... 49 cm... 50 cm.

In some embodiments, channels of a microfluidic device may be of any suitable size and dimensions. In some embodiments, channels are so dimensioned to allow the appropriate solutions to efficiently traverse and/or occupy the microfluidic channels. In some embodiments, the length of a channel is proportional to the length of the device. In some embodiments, the length of a channel is proportional to the length of the membrane and/or gel with which it is configured for use. In some embodiments, channels are between 1 cm and 50 cm in length (e.g., 1 cm... 2 cm... 3 cm... 4 cm... 5 cm... 6 cm... 7 cm... 8 cm... 9 cm... 10 cm... 11 cm... 12 cm... 13 cm... 14 cm... 15 cm...16 cm... 17 cm... 18 cm... 19 cm... 20 cm... 21 cm... 22 cm... 23 cm... 24 cm... 25 cm... 26 cm... 27 cm... 28 cm... 29 cm... 30 cm... 31 cm... 32 cm... 33 cm... 34 cm... 35 cm... 36 cm... 37 cm... 38 cm... 39 cm... 40 cm... 41 cm... 42 cm... 43 cm... 44 cm... 45 cm... 46 cm... 47 cm... 48 cm... 49 cm... 50 cm). In some embodiments, a channel is of appropriate width and/or depth to allow the necessary solutions (e.g., antibody solutions) to flow/traverse, occupy, and/or fill the channel. In some embodiments, the width of a channel is optimized to provide the desired number of channels in a set of a particular width. In some embodiments, as channels are narrowed to accommodate more lanes per set, channels are deepened to provide the necessary channel volumes. In some embodiments, a set of channels ranges in width from 1 mm to 50 cm (e.g., 1 mm... 2 mm... 3 mm... 4 mm... 5 mm... 6 mm... 7 mm... 8 m... 9 mm... 1 cm... 2 cm... 3 cm... 4 cm... 5 cm... 6 cm... 7 cm... 8 cm... 9 cm... 10 cm... 11 cm... 12 cm... 13 cm... 14 cm... 15 cm...16 cm... 17 cm... 18 cm... 19 cm... 20 cm... 21 cm... 22 cm... 23 cm... 24 cm... 25 cm... 26 cm... 27 cm... 28 cm... 29 cm... 30 cm... 31 cm... 32 cm... 33 cm... 34 cm... 35 cm... 36 cm... 37 cm... 38 cm... 39 cm... 40 cm... 41 cm... 42 cm... 43 cm... 44 cm... 45 cm... 46 cm... 47 cm... 48 cm... 49 cm... 50 cm). In some embodiments, set width is configured to match the lane width of a gel and/or membrane. In some embodiments, a set of microchannels comprises one or more channels (e.g., 1, 2, 3, 4, 5, 6, 7, 8, 9, 10... 20... 30... 40... 50... 100... 500, etc.). In some embodiments, the number of channels per set is dependent upon the width of the set, the spacing between channels, and the width of the channels (e.g., which may be dependent upon the type of solution to be used within the channels). In some embodiments, channels widths range from 50 µm to 500 µm (e.g., 50 µm, 60 µm, 70 µm, 80 µm, 90 µm, 100 µm, 110 µm, 120 µm, 130 µm, 140 µm, 150 µm, 160 µm, 170 µm, 180 µm, 190 µm, 200 µm... 250 µm... 300 µm... 350 µm... 400 µm... 450 µm... 500 µm). In some embodiments, channel widths range from 100 µm to 200 µm (e.g., 125 µm to 175 µm, 140 µm to 160 µm, about 150 µm, 150 µm). In some embodiments, channel depths range from 25 µm to 200 µm (e.g., 25 µm, 30 µm, 40 µm, 50 µm, 60 µm, 70 µm, 80 µm, 90 µm, 100 µm, 110 µm, 120 µm, 130 µm, 140 µm, 150 µm, 160 µm, 170 µm, 180 µm, 190 µm, 200 µm). In some embodiments, channel widths range from 75 µm to 125 µm (e.g., 90 µm to 110 µm, about 100 µm, 100 µm). In some embodiments, channels accomdate between 0.1 µl and 5 µl of solution per channel (e.g., 0.1 µl, 0.2 µl, 0.3 µl, 0.4 µl, 0.5 µl, 0.6 µl, 0.7 µl, 0.8 µl, 0.9 µl, 1.0 µl, 1.1 µl, 1.2 µl, 1.3 µl, 1.4 µl, 1.5 µl, 1.6 µl, 1.7 µl, 1.8 µl, 1.9 µl, 2 µl... 3 µl... 4 µl... 5 µl).

In some embodiments, microfluidic devices are provided as part of a kit along with one or more of appropriately sized membranes, appropriately sized gel with lanes configured to match channels of the device, antibody solutions, buffer, activation solution, comb for pouring gels with lanes configured to match channels of the device, instructions, etc. In some embodiments, a device or kit is optimized for a particular assay. In some embodiments, a kit is provided with antibodies and lane configuration for performing a specific assay.

In some embodiments, the channel sets and/or microfluidic channels are not consistent across the device. Channels on a single device may vary across a single device according to one or more of set width, number of microchannels per set, microchannel width, spacing, etc.

### B. Method

In some embodiments, devices of the present invention are utilized to perform immunoblot experiments, using any suitable techniques and reagents. \

An exemplary procedure for immunoblotting with a device of the present invention is as follows. A protein-containing sample is loaded into the wells of a gel (e.g., polyacrylamide gel) and proteins present in the sample are separated by gel electrophoresis (e.g., native gel electrophoresis, SDS-PAGE, etc.). A membrane (e.g., PVDF membrane) is placed atop the gel to allow transfer of the proteins onto the membrane. The membrane is then placed on a glass slide. A microfluidic device is placed on top of membrane, and the microfluidic channels are aligned with the protein lanes. Blocking buffer (e.g., Tween-20 and BSA) is optionally injected in each of the channels. In embodiments with a blocking step, the inlets/outlets of the channels are covered (e.g., with tape) to minimize evaporation during incubation. Antibodies are injected into the appropriate channels (e.g., different antibodies in each microchannel of a set). The inlets/outlets are covered (e.g., with tape) to minimize evaporation during incubation. Following incubation (e.g., 1 hours), the microfluidic device is removed and the membrane is transferred to a solution of an appropriate secondary antibody (e.g., the membrane is left in this solution for 1 hour, or until the membrane is completely wet). The membrane is removed from the secondary antibody solution and washed with fresh blocking buffer (e.g., 3 minutes, 5 times each). The membrane is transferred to a developing solution (e.g., 30 minutes), and the membrane is removed and rinsed with DI water. The membrane is then allowed to dry. In some embodiments, any or all of the above steps may be altered for an alternative procedure or to accommodate a specific use. For example, in some embodiments, the device is placed onto the membrane with antibodies pre-loaded into the channels. In other embodiments, the blocking, secondary antibody solution, washing, and developing solutions are applied through the microfluidic channels. In some embodiments, a sample is placed on a membrane directly or from a surface other than a gel (e.g., microtiter plate, array, etc.).

In some embodiments, the same sample under the same conditions is run on multiple wells of a gel. In such cases, the microfluidic channels of multiple sets of channels may all be loaded with different antibodies to interrogate the same sample. In other embodiments, different samples (or the same sample under different conditions) are run on two or more lanes of a gel. In such cases, the various channel sets may be loaded identically, to probe each sample with the same set of antibodies.

In some embodiments, the device and/or membrane comprise hydrophobic surfaces (e.g., PDMS and PVDF). Experiments conducted during development of embodiments of the present invention revealed that it is challenging to achieve a suitable seal between the device and membrane. In some embodiments, to overcome this challenge, an activation step (e.g., of the device and/or of the membrane) is performed to enhance the seal between the device and membrane. In some embodiments, the seal is activated through the microfluidic channels. In some embodiments, an activating solution is applied to the membrane-contacting surface of the device. In some embodiments, an activating solution is applied to the membrane. In some embodiments, the activation takes advantage of the hydrophobic/hydrophilic differences between the PDMS/PVDF surfaces and the aqueous solutions used.

In some embodiments, solutions are added to microfluidic channels through any suitable structure or method. In some embodiments, solutions are injected into channels. In some embodiments, solutions are injected into reservoirs at one end of the channels. In some embodiments, pumps and/or valves are not required for solution addition. In some embodiments, a needle (e.g., 10-40 gauge (e.g., 12 gauge... 16 gauge... 20 gauge... 24 gauge... 27.5 gauge... 30 gauge... 36 gauge... 40 gauge) is used to introduce solutions (e.g., antibody solutions) into the channel. In some embodiments, introduction of solutions from an appropriately sized needle (e.g., 27.5 gauge) into an appropriately sized reservoir produces a "tamponade" effect around the needle that greatly enhances the seal and facilitates filling of the microfluidic channel.

### C. Fabrication

In some embodiments, devices of the present invention are fabricated using microfluidics fabrication techniques that are known in the field. A variety of exemplary fabrication methods are described in Fiorini and Chiu, 2005, "Disposable microfluidic devices: fabrication, function, and application" Biotechniques 38:429-46; Beebe et al., 2000, "Microfluidic tectonics: a comprehensive construction platform for microfluidic systems." Proc. Natl. Acad. Sci. USA 97:13488-13493; Rossier et al., 2002, "Plasma etched polymer microelectrochemical systems" Lab Chip 2:145-150; Becker et al., 2002, "Polymer microfluidic devices" Talanta 56:267-287; Becker et al., 2000, "Polymer micro fabrication methods for microfluidic analytical applications" Electrophoresis 21:12-26; U.S. Pat. No. 6,767,706 B2, e.g., Section 6.8 "Microfabrication of a Silicon Device"; Terry et al., 1979, A Gas Chromatography Air Analyzer Fabricated on a Silicon Wafer, IEEE Trans. on Electron Devices, v. ED-26, pp. 1880-1886; Berg et al., 1994, Micro Total Analysis Systems, New York, Kluwer; Webster et al., 1996, Monolithic Capillary Gel Electrophoresis Stage with On-Chip Detector in International Conference On Micro Electromechanical Systems, MEMS 96, pp. 491496; and Mastrangelo et al., 1989, Vacuum-Sealed Silicon Micromachined Incandescent Light Source, in Intl. Electron Devices Meeting, IDEM 89, pp. 503-506. In some embodiments, the methods used in fabrication of a microfluidic device may vary with the materials used, and include soft lithography methods, microassembly, bulk micromachining methods, surface micro-machining methods, standard lithographic methods, wet etching, reactive ion etching, plasma etching, stereolithography and laser chemical three-dimensional writing methods, modular assembly methods, replica molding methods, injection molding methods, hot molding methods, laser ablation methods, combinations of methods, and other methods known in the art or developed in the future.

An exemplary method of device fabrication is as follows. A computer aided design (CAD) document is generated that provides the design specifications for the device (e.g., device dimensions, channel dimensions, channel spacing, number of channels per set, number of sets of channels, channel shape, presence of reservoirs, reservoir shape/size, etc.). From the CAD specification, a mask (e.g., glass, polymer, etc.) is produced that provides a tangible design of the device. From the mask, a mold (e.g., silicon wafer mold) is produced by pouring uncured polymer (e.g., polydimethylsiloxane (PDMS)) into the mold, followed by heating of the mask and polymer to cure the polymer (e.g., polymerize, crosslink, etc.), yielding a solid microfluidic device. In some embodiments, a single mask is reused to produce multiple (e.g., 10, 100, 1000, 10,000, or more) molds. In some embodiments, a single mold is reused to produce multiple (e.g., 10, 100, 1000, 10,000, or more) devices. In some embodiments, microfluidic devices are fabricated from polymethylsixoxane (PDMS) using soft-lithography techniques; although, and suitable polymer(s) and techniques are within the scope of the present invention.

### D. Antibodies/Reagents

In some embodiments, solutions for use with the devices described herein are be configured to fill microfluidic channels is an efficient and reproducible manner. To this end, solutions should have appropriate viscosity, hydrophilicity, etc. Experiments were conduted during development of embodiments of the present invention to develop solutions with appropriate characteristic for deployment in the microfluicid channels of the devices described herein. In some embodiments, solutions comprise one or more surfactants, detergents, emulsifiers, solubilizers, etc. to provide acceptable/optimal filling the microfluidic channels in a fast and reproducible manner. In some embodiments, solutions comprise one or more of: ammonium lauryl sulfate, sodium lauryl sulfate (SDS, sodium dodecyl sulfate), sodium laureth sulfate, sodium myreth sulfate, dioctyl sodium sulfosuccinate, perfluorooctanesulfonate (PFOS), perfluorobutanesulfonate, linear alkylbenzene sulfonates (LABs), sodium stearate, sodium lauroyl sarcosinate, perfluorononanoate, perfluorooctanoate, alkyltrimethylammonium salts (e.g., cetyl trimethylammonium bromide), cetylpyridinium chloride (CPC), benzalkonium chloride (BAC), benzethonium chloride (BZT), 5-Bromo-5-nitro-1,3-dioxane, dimethyldioctadecylammonium chloride, cetrimonium bromide, dioctadecyldimethylammonium bromide (DODAB), CHAPS, cocamidopropyl hydroxysultaine, lecithin, polyoxyethylene glycol alkyl ethers, polyoxypropylene glycol alkyl ethers, glucoside alkyl ethers, polyoxyethylene glycol octylphenol ethers (e.g., Triton X-100), Polyoxyethylene glycol alkylphenol ethers, glycerol alkyl esters, polyoxyethylene glycol sorbitan alkyl esters (e.g., Polysorbate (e.g., Polysorbate 20, Polysorbate 40, Polysorbate 60, Polysorbate 80, etc.)), sorbitan alkyl esters, cocamide MEA, cocamide DEA, dodecyldimethylamine oxide, block copolymers of polyethylene glycol and polypropylene glycol, polyethoxylated tallow amine (POEA), etc.

In some embodiments, solutions (e.g., antibody solutions, activating solutions, blocking solutions, washing solutions, etc.) comprise tailored concentrations of Tween (e.g., Tween-20) and bovine serum albumin (BSA). In some embodiments, useful concentrations of Tween (e.g., Tween-20) and BSA are utilized to provide for efficient flowing of solutions for the length of the microfluidic channels. In some embodiments, useful concentrations of Tween (e.g., Tween-20) and BSA are utilized to provide activation/wetting of the membranes through the microfluidic channels. In some embodiments, solutions comprise between 0.01% and 5% BSA (e.g., 0.01%... 0.02%... 0.05%... 0.1%... 0.2%... 0.5%... 1%... 2%... 5%). In some embodiments, solutions comprise between 0.01% and 5% Tween (e.g., Tween20) (e.g., 0.01%... 0.02%... 0.05%... 0.1%... 0.2%... 0.5%... 1%... 2%... 5%). In some embodiments, solutions comprise about 0.1% BSA and about 0.1% Tween20. In some embodiments, solutions comprise 0.1% BSA and 0.1% Tween20.

In some embodiments, solutions (e.g., blocking, activating, washing, antibody, etc.) comprise appropriate salts, buffers, metals, etc. as would be understood by one of skill in the art.

In some embodiments, antibody binding to target proteins are visualized and/or detected through the use of a detectable/observable moieties and/or labels. Suitable labels and/or moieties are detected spectroscopic, photochemical, biochemical, immunochemical, electrical, optical or chemical means. In some embodiments, primary or secondary antibodies are linked to a detection moiety. In some embodiments, detection is performed enzymatically using, for example alkaline phosphatase or horseradish peroxidase. Some embodiments, utilize fluorescent detection. Fluorophores contemplated to be useful in the present disclosure include Alexa dyes (e.g., Alexa 350, Alexa 430, etc.), AMCA, BODIPY 630/650, BODIPY 650/665, BODIPY-FL, BODIPY-R6G, BODIPY-TMR, BODIPY-TRX, Cascade Blue, Cy2, Cy3, Cy5, 6-FAM, Fluorescein, HEX, 6-JOE, Oregon Green 488, Oregon Green 500, Oregon Green 514, Pacific Blue, REG, Rhodamine Green, Rhodamine Red, ROX, TAMRA, TET, Tetramethylrhodamine, Texas Red, etc. One of skill in the art will recognize that these and other detection moieties not mentioned herein can be used with success in various embodiments.

Any antibodies, antibody fragments, or other molecules capable of specifically and stably binding a target, analyte, or antigen find use as the primary and secondary antibodies described herein (e.g., dendrimer, nucleic acid, affinity tag, etc.). Although the embodiments described herein have typically utilized antibodies (e.g., primary antibodies, secondary antibodies) for interrogating the protein contents of a sample, the present invention is not limited to the use of antibodies. Embodiments should be read broadly as also encompassing other binding molecules.

### E. Applications

Embodiments of the present invention find use in any application in which traditional Western blotting has been traditionally used. Moreover, due to removal of the cost, time, reagent, throughput, and multiplexability limitations of Western blotting, devices and method described herein find use in additional research, clinical, diagnostic, and epidemiological application to which traditiona Western blotting is insufficient. For example, rapid, parallel profiling of signaling pathways has been a long sought after goal in biological sciences and clinical medicine. To fully understand the dynamics of these signaling pathways, their protein components must be profiled. These components are typically profiled with protein immunoblotting. Although traditional protein immunoblotting is a powerful technique, it has several limitations including the large sample requirements, high antibody amounts, and limitations in assay throughput. The microfluidic immunoblotting devices of the present invention provide rapid, parallel profiling of the protein components of signaling pathways. The utility of the present devices and methods have been demonstrated by profiling common inflammatory signaling pathways (e.g., NFκB, JAK-STAT, and MAPK) in cell models and clinical samples. Experiments conducted during development of embodiments of the present invention demonstrate that microfluidic immunoblotting devices can profile endogenous proteins with accuracy similar to that of traditional protein immunoblotting but with 2800-fold less antibody and the ability to multiplex. Additionally, the present microfluidic device interfaces with commonly available immunoblotting equipment and is compatible with existing protein detection methodologies.

### EXPERIMENTAL

Over the past decade, inflammation has been recognized as a driver of chronic diseases including cancer and heart disease (Saleh and Trinchieri 2011; Hansson 2005). Although many regulatory steps are involved, protein modifications are defining features of inflammatory responses (Anderson 2010; Anderson 2008; Akira and Takeda 2004). As appreciation of the importance of protein activation in inflammatory responses has grown, there is an increasing need for more robust techniques to monitor these dynamic responses.

### Example 1

### Materials and Methods

### Microfluidic protein immunoblotting

After gel electrophoresis, the gels were blotted onto a PVDF membrane (Invitrogen) for 1 hour at 30V. The electroblotted PVDF membrane was dried overnight before proceeding to microfluidic immunoblotting. The microfluidic assembly was created by sandwiching the electroblotted PVDF membrane between a glass support and the PDMS microfluidic device. The PDMS microfluidic device was aligned to the ladder to ensure accurate channel placement over the sample lanes. To complete the assembly, two glass slides were placed on top of the PDMS device for further support. The spacing between the two glass slides was approximately 0.5mm and was used as the injection site. The activating and antibody solutions were injected using a 1ml syringe with 27G ½ needles. The PVDF membrane was activated through the microfluidic channels using a 0.1% BSA and 0.1% Tween20 TBS solution. Primary antibodies were injected through the microfluidic channels and incubated for 1 hour. Both the primary and secondary antibody dilutions were prepared in 0.1% BSA and 0.1% Tween20 TBS solutions.

Following the primary antibody incubation period, the microfluidic device was removed and the membrane was washed, blocked for 1 hour in 0.1% BSA and 0.1% Tween20 TBS, and then stained with the secondary antibody for 1 hour prior to chemiluminescent detection with either alkaline phosphatase or horseradish peroxidase.

### Gel electrophoresis and protein immunoblotting

For peripheral blood mononuclear cells (PBMCs), healthy individuals who agreed to participate in this institutional review board study were provided with written informed consent. PBMCs were isolated using BD Vacutainer CPT Cell Preparation Tubes with Sodium Citrate. Following isolation, PBMC cell lysates were collected using RIPA buffer as described below. The murine macrophage cell line (RAW264.7) was purchased from American Type Culture Collection (ATCC; Manassas, VA) and cultured in RPMI medium (Invitrogen; Carlsbad, CA) supplemented with penicillin (100 U/ml), streptomycin (100 µg/ml) and 10% fetal bovine serum (Invitrogen) at a density of 3.0 X 106. After the cells were allowed to adhere for 4 to 6 hours, the media was changed to RPMI media supplemented with 0.5% FBS, penicillin (100 U/ml), streptomycin (100 µg/ml), and incubated overnight (14-18 h). Endotoxin stimulation was initiated by incubating cells in 0.5% FBS medium containing lipopolysaccharide (LPS) from Escherichia coli O55:B5 (Sigma-Aldridge; St Louis, MO) at a concentration of 100 ng/ml. Cells were then harvested and lysed using RIPA buffer containing 25 mM Tris-HCl, pH 7.6, 150 mM NaCl, 1% Nonidet P-40, 1% sodium deoxycholate, 1 mM Na3VO4, and 10 mM NaF (phosphatase inhibitors) and 0.1% SDS with 10 µl of Halt Protease Inhibitor Cocktail (Pierce, Rockford, IL) added for each 1 ml of buffer. Samples were separated using SDS-PAGE electrophoresis and transferred to a PVDF membrane. PVDF membranes were then probed with rabbit antibodies against active-JNK (phospho-JNK1/2; Promega; Madison, WI), active-MAPK (phospho-ERK; Promega), and ERK-1 (Santa Cruz Biotechnology; Santa Cruz, CA), NF-κB p65 (Cell Signaling Technology; Beverly, MA), STAT3 (sc-483, Santa Cruz Biotechnology), and phospho-STAT3 (Cell Signaling Technology). Horseradish peroxidase or alkaline phosphatase goat anti-rabbit secondary antibodies (Thermo Scientific) were used at a dilution of 1 :20000.

### Example 2

### Results

### Design and interface of microfluidic device

Poly(dimethysiloxane) (PDMS) microfluidic devices were designed to interface with existing immunoblotting equipment. The devices, which were approximately 8 cm long and 6 cm wide, consisted of 5 lanes spaced at intervals corresponding to the space between the protein lanes in a traditional Western blot. Within each lane resided 3 microfluidic channels (6 cm long, 150 µM wide, 100 µM deep.).

The devices were fabricated using standard soft-lithography techniques.(Wu et al. 2003) Briefly, a transparency mask was printed using a CAD file that contained the design of the microfluidic device (CAD/Art Services, Inc., Bandon, OR). Soft-lithography was used to fabricate a silicon master mold from the transparency mask. The microfluidic devices were formed by mixing the pre-polymer and curing agent at a mass ratio of 12:1 (Sylgard 184 Silicone Elastomer Kit, Dow Corning) and then pouring the mixture on to the silicon master. The uncured device was degassed to remove any air bubbles formed during mixing before curing at 60° C for 1 hour.

The approach consists of three stages including gel electrophoresis, transfer to a PVDF membrane, and immunoblotting for target proteins using the microfluidic device **(****Figure 1****).** The first two stages are similar to traditional immunoblotting that allows our microfluidic device to easily interface with existing equipment.

Following the separation and transfer of samples, the PVDF membrane was dried. The PVDF membrane was then sandwiched between a glass support and the PDMS microfluidic device. The PVDF membrane was activated by injecting blocking solution through the microfluidic and allowing it to sit for 20 minutes. After activating the PVDF membrane, the primary antibody was injected through the microfluidic channels and incubated for 1 hour. After incubation with the primary antibody, the microfluidic device was removed and the PVDF membrane was washed and blocked prior to proceeding to the detection phase. To detect the primary antibody, the entire PVDF membrane was incubated with the appropriate secondary antibody and detected with either alkaline phosphatase or horseradish peroxidase chemiluminescent methodologies.

### Comparison of traditional and microfluidic immunoblotting

To directly assess the accuracy of our microfluidic immunoblotting device compared to traditional immunoblotting, peripheral blood monocytes (PBMC) were collected from healthy volunteers and isolated cell lysates as previously described. 5 and 0.5 micrograms of protein were separated using gel electrophoresis followed by transfer to the PVDF membrane. Using a primary antibody to RelA (p65), a member of the NF-κB transcriptional family, the signals between microfluidic and traditional immunoblotting were compared at two different primary antibody dilutions and protein loading amounts **(****Figure 2****).** For the two loading amounts and antibody dilutions, both approaches demonstrated similar signals for p65. Importantly, with the microfluidic device obtained similar signal intensities using the same antibody dilutions as used with the traditional immunoblotting. For the PVDF membrane size used in these experiments, a traditional immunoblotting assay for 1 protein requires approximately 10 mL of primary antibody solution. Based on the dimensions of a microfluidic channel (0.015, 0.010, and 6.000 cm), each channel requires approximately 0.0009 mL of primary antibody solution. Using the microfluidic device, to assay a PVDF membrane for 1 protein requires 0.0036 mL of primary antibody solution (4 lanes *0.0009 mL of antibody/lane = 0.0036 mL antibody per protein). Even with conservative assumptions and ignoring its multiplexing capacity, microfluidic immunoblotting translates into approximately 2800-fold reduction in antibody amounts compared with traditional protein immunoblotting.

### Monitoring inflammatory pathway activation and protein modifications

Inflammatory pathways are often regulated by posttranslational modifications of signaling proteins (Medzhitov 2008, 2010). Monitoring these protein modifications is essential to understand the dynamics of inflammatory responses. Similar to the NF-κB transcriptional family, the signal transducer and activator of transcription (STAT) family is a key mediator of inflammatory responses (Ho et al. 2009). In response to cytokine and growth factors, STAT family members are phosphorylated by receptor-associated kinases (Hu et al. 2007; O'Sullivan et al. 2007). Protein phosphorylation is typically monitored with protein immunoblotting but also requires parallel monitoring of total protein levels. In the context of STAT3 activation, protein immunoblotting must be used to monitor both phosphorylated and total STAT3 levels from the same sample. These types of experiments are well suited for microfluidic immunoblotting because the microfluidic device enables the simultaneous profiling of phosphorylated and total protein from the same sample lane. To evaluate our microfluidic device in this capacity, RAW264.7 cells, a macrophage cell line, were stimulated with lipopolysaccharide (LPS) (100 ng/ml) for 24 hours. After 24 hours, the cell lysates were collected and probed for phospho-STAT3 and STAT3 using the microfluidic device and traditional immunoblotting. Similar to traditional immunoblotting, microfluidic immunoblotting detected phospho-STAT3 in LPS stimulated macrophages consistent with inflammatory activation of STAT signaling **(****Figure 3****).**

These results suggest that the microfluidic device can be used to monitor posttranslational modifications associated with inflammatory responses. The ability to monitor multiple proteins from the same sample without the need for membrane stripping greatly improves the assay throughput and strengthens conclusions that can be drawn from these types of immunoblotting experiments.

### Monitoring multicomponent, inflammatory pathways

The mitogen activated protein kinase (MAPK) pathway is another pathway that coordinates inflammatory responses (Chang and Karin 2001). More specifically, MAPKs are serine/threonine-specific protein kinases that transduce extracellular signals and regulate diverse cellular responses including cell proliferation, differentiation, and apoptosis. One of the difficulties in monitoring this pathway is that it has multiple protein components with different patterns of activation. Because of these complexities, monitoring the MAPK pathway with traditional immunoblotting is time and resource intensive.

One of the unique aspects of the present micro fluidic immunoblotting approach is the interface between the PDMS microfluidic device and the PVDF membrane. To achieve an efficient interface, the composition and the duration of the activation solution are critical parameters. In order to maintain an efficient seal between the PDMS device and the PVDF membrane, the initial blocking step that is used with traditional immunoblotting was eliminated. However, because of the cross-reactivity of some antibodies and the elimination of the blocking step, there was the potential for more non-specific signals with microfluidic immunoblotting compared with traditional immunoblotting that would be compounded by the complexity of the MAPK signaling pathway. To evaluate the ability of our microfluidic device to monitor the multicomponent, MAPK pathway, RAW264.7 cells were incubated with LPS (100 ng/ml) for 45 minutes and collected cell lysates as described above. The samples were probed for phospho-JNK, phospho-ERK, and total ERK with traditional and microfluidic protein immunoblotting **(****Figure 4****).** In contrast to traditional immunoblotting that required three different PVDF membranes, the microfluidic approach allowed simultaneous monitoring of these protein components with only two sample lanes from half of a PVDF membrane. By simultaneously probing for total ERK from the same sample, time, resources, and variability were minimized compared with traditional immunoblotting. Importantly, there was equivalent background with both approaches even with the elimination of the blocking step in the microfluidic immunoblotting approach.

The flexibility of the microfluidic immunoblotting technique was evaluated with other commonly used chemiluminescent detection methods. Both horseradish peroxidase and alkaline phosphatase have excellent sensitivity. However, this higher sensitivity also makes them more susceptible to background noise. A microfluidic immunoblot was run using the same cell lysates and MAPK antibodies as above. Then used a horseradish peroxidase secondary antibody was used to detect the primary antibodies. The efficacy of HRP detection was equivalent to alkaline phosphatase (AP) detection using the microfluidic immunoblotting device **(****Figure 4****)** further expanding the utility of this approach.

### LITERATURE REFERENCES

Akira S, Takeda K (2004) Toll-like receptor signalling. Nat Rev Immunol 4 (7):499-511.
Anderson P (2008) Post-transcriptional control of cytokine production. Nat Immunol 9 (4):353-359.
Anderson P (2010) Post-transcriptional regulons coordinate the initiation and resolution of inflammation. Nat Rev Immunol 10 (1):24-35.
Anderson P, et al. (2011) Western blotting using capillary electrophoresis. Anal Chem 83(4): 1350-1355.
Chang L, Karin M (2001) Mammalian MAP kinase signalling cascades. Nature 410 (6824):37-40.
Chang L, et al. (2013) Profiling inflammatory responses with microfluidic immunoblotting. PLOS ONE 8(11): 1-11.
Chen X, Kapil MA, Hughes AJ, Herr AE (2011) Single-microchannel, multistep assay reports protein size and immunoaffinity. Anal Chem 83 (17):6573-6579.
Ciaccio MF, Wagner JP, Chuu CP, Lauffenburger DA, Jones RB (2010) Systems analysis of EGF receptor signaling dynamics with microwestern arrays. Nat Methods 7 (2):148-155.
Geng T, Bao N, Litt MD, Glaros TG, Li L, Lu C (2011) Histone modification analysis by chromatin immunoprecipitation from a low number of cells on a microfluidic platform. Lab Chip 11 (17):2842-2848.
Hansson GK (2005) Inflammation, atherosclerosis, and coronary artery disease. N Engl J Med 352 (16):1685-1695. doi:352/16/1685 [pii]
He M, Herr AE (2009) Microfluidic polyacrylamide gel electrophoresis with in situ immunoblotting for native protein analysis. Anal Chem 81 (19):8177-8184.
Ho MK, Su Y, Yeung WW, Wong YH (2009) Regulation of transcription factors by heterotrimeric G proteins. Curr Mol Pharmacol 2 (1):19-31
Hu X, Chen J, Wang L, Ivashkiv LB (2007) Crosstalk among Jak-STAT, Toll-like receptor, and ITAM dependent pathways in macrophage activation. J Leukoc Biol 82 (2):237-243.
Hughes AJ, Herr AE (2012) Microfluidic Western blotting. Proc Natl Acad Sci USA 109 (52):21450-21455.
Medzhitov R (2008) Origin and physiological roles of inflammation. Nature 454 (7203):428-435.
Medzhitov R (2010) Inflammation 2010: new adventures of an old flame. Cell 140 (6):771-776. doi:S0092.
O'Sullivan LA, Liongue C, Lewis RS, Stephenson SE, Ward AC (2007) Cytokine receptor signaling through the Jak-Stat-Socs pathway in disease. Mol Immunol 44 (10):2497-2506.
Pan W, Chen W, Jiang X (2010) Microfluidic Western blot. Anal Chem 82 (10):3974-3976.
Pimkova K, Bockova M, Hegnerova K, Suttnar J, Cermak J, Homola J, Dyr JE (2011) Surface plasmon resonance biosensor for the detection of VEGFR-1-a protein marker of myelodysplastic syndromes. Anal Bioanal Chem.
Saleh M, Trinchieri G (2011) Innate immune mechanisms of colitis and colitis-associated colorectal cancer. Nat Rev Immunol 11 (1):9-20.
Tia SQ, He M, Kim D, Herr AE (2011) Multianalyte On-Chip Native Western Blotting. Anal Chem.
Towbin H, Staehelin T, Gordon J (1979) Electrophoretic transfer of proteins from polyacrylamide gels to nitrocellulose sheets: procedure and some applications. Proc Natl Acad Sci U S A 76 (9):4350-4354
Wu H, Odom TW, Chiu DT, Whitesides GM (2003) Fabrication of complex three-dimensional microchannel systems in PDMS. J Am Chem Soc 125 (2):554-559.
Wu Y, Li Q, Chen XZ (2007) Detecting protein-protein interactions by Far western blotting. Nat Protoc 2 (12):3278-3284.
Zubair A, Burbelo PD, Vincent LG, Iadarola MJ, Smith PD, Morgan NY (2011) Microfluidic LIPS for serum antibody detection: demonstration of a rapid test for HSV-2 infection. Biomed Microdevices 13 (6):1053-1062.

### PATENT REFERENCES

WO 2011/106693 (UNIVERSITY OF MICHIGAN [US])
US 2011/312804 (GENEASYS [AU])
US 2011/177618 (AMY E. HERR ET AL. [US])
(US 2012/211373 (UNIVERSITY OF MICHIGAN [US])
US2012/202219 (H.D.AGNEW & S.M. PITRAM [US])
WO 2007/060580 (KONINKLIJKE PHILIPS ELECTRONICS NV [NL])

## Claims

1. A microfluidic immunoblotting device comprising a flat membrane-contacting surface and a plurality of non-connected parallel microfluidic channels, wherein the entire length of said microfluidic channels are open to the membrane-contacting surface.

2. The microfluidic immunoblotting device of claim 1, wherein said microfluidic channels are spatially grouped into two or more channel sets.

3. The microfluidic immunoblotting device of claim 2, wherein the distance separating microfluidic channels with a channel set is less than the distance between channel sets.

4. The microfluidic immunoblotting device of claim 1, wherein each of the microfluidic channels comprises a reservoir at one end of the microfluidic channel.

5. The microfluidic immunoblotting device of claim 1, wherein each of the microfluidic channels is 100-200 µm wide.

6. The microfluidic immunoblotting device of claim 1, wherein each of the microfluidic channels is 50-150 µm deep.

7. The microfluidic immunoblotting device of claim 1, wherein said device comprises polydimethylsiloxane (PDMS).

8. A method of immunoblotting comprising applying antibody solutions to a membrane using a device of one of claims 1-7.

9. A system comprising a device of one of claims 1-7 and a membrane, further comprising a activating solution between said device and said membrane.

10. The system of claim 9, wherein said membrane comprises membrane nitrocellulose or polyvinylidene fluoride (PVDF).

11. The system of claim 9, wherein said membrane comprises peptides, polypeptides, or proteins on or within it.

12. The system of claim 9, further comprising one or more antibody-containing solutions within the microfluidic channels.

13. The system of claim 12, wherein each microfluidic channel of a channel set comprises a different antibody-containing solution or each channel set comprises the same combination of antibody-containing solutions.

14. The system of claim 12, wherein each microfluidic channel comprises a different antibody-containing solution.

15. A method of immunoblotting comprising applying antibody solutions to a membrane using system of one of claims 9-14.

## Patentansprüche

1. Mikrofluidische Immunoblotting-Vorrichtung, umfassend eine ebene membrankontaktierende Oberfläche und mehrere nicht verbundene parallele mikrofluidische Kanäle, wobei die mikrofluidischen Kanäle auf ihrer gesamten Länge zur membrankontaktierenden Oberfläche offen sind.

2. Mikrofluidische Immunoblotting-Vorrichtung nach Anspruch 1, wobei die mikrofluidischen Kanäle räumlich in zwei oder mehr Kanalsätzen gruppiert sind.

3. Mikrofluidische Immunoblotting-Vorrichtung nach Anspruch 2, wobei der Trennabstand von mikrofluidischen Kanälen bei einem Kanalsatz kleiner als der Abstand zwischen Kanalsätzen ist.

4. Mikrofluidische Immunoblotting-Vorrichtung nach Anspruch 1, wobei die mikrofluidischen Kanäle jeweils ein Reservoir an einem Ende des mikrofluidischen Kanals umfassen.

5. Mikrofluidische Immunoblotting-Vorrichtung nach Anspruch 1, wobei die mikrofluidischen Kanäle jeweils eine Breite von 100-200 µm aufweisen.

6. Mikrofluidische Immunoblotting-Vorrichtung nach Anspruch 1, wobei die mikrofluidischen Kanäle jeweils eine Tiefe von 50-150 µm aufweisen.

7. Mikrofluidische Immunoblotting-Vorrichtung nach Anspruch 1, wobei die Vorrichtung Polydimethylsiloxan (PDMS) umfasst.

8. Immunoblotting-Verfahren, umfassend Auftragen von Antikörperlösungen auf eine Membran unter Verwendung einer Vorrichtung nach einem der Ansprüche 1-7.

9. System, umfassend eine Vorrichtung nach einem der Ansprüche 1-7 und eine Membran, ferner umfassend eine Aktivierungslösung zwischen der Vorrichtung und der Membran.

10. System nach Anspruch 9, wobei die Membran Nitrocellulose oder Polyvinylidenfluorid (PVDF) umfasst.

11. System nach Anspruch 9, wobei die Membran Peptide, Polypeptide oder Proteine auf oder in ihr umfasst.

12. System nach Anspruch 9, ferner umfassend eine oder mehrere antikörperhaltige Lösungen in den mikrofluidischen Kanälen.

13. System nach Anspruch 12, wobei die mikrofluidischen Kanäle eines Kanalsatzes jeweils eine unterschiedliche antikörperhaltige Lösung umfassen oder die Kanalsätze jeweils die gleiche Kombination antikörperhaltiger Lösungen umfassen.

14. System nach Anspruch 12, wobei die mikrofluidischen Kanäle jeweils eine unterschiedliche antikörperhaltige Lösung umfassen.

15. Immunoblotting-Verfahren, umfassend Auftragen von Antikörperlösungen auf eine Membran unter Verwendung eines Systems nach einem der Ansprüche 9-14.

## Revendications

1. Dispositif microfluidique d'immunobuvardage comprenant une surface de contact avec une membrane plate et une pluralité de canaux microfluidiques parallèles non raccordés, dans lequel la totalité de la longueur desdits canaux microfluidiques est ouverte sur la surface en contact avec la membrane.

2. Dispositif microfluidique d'immunobuvardage selon la revendication 1, dans lequel lesdits canaux microfluidiques sont groupés dans l'espace en deux ensembles ou plus de canaux.

3. Dispositif microfluidique d'immunobuvardage selon la revendication 2, dans lequel la distance séparant les canaux microfluidiques d'un ensemble de canaux est inférieure à la distance entre les ensembles de canaux.

4. Dispositif microfluidique d'immunobuvardage selon la revendication 1, dans lequel chacun des canaux microfluidiques comprend un réservoir à une extrémité du canal microfluidique.

5. Dispositif microfluidique d'immunobuvardage selon la revendication 1, dans lequel chacun des canaux microfluidiques a une largeur de 100-200 µm.

6. Dispositif microfluidique d'immunobuvardage selon la revendication 1, dans lequel chacun des canaux microfluidiques a une profondeur de 50-150 µm.

7. Dispositif microfluidique d'immunobuvardage selon la revendication 1, dans lequel ledit dispositif comprend du polydiméthylsiloxane (PDMS).

8. Procédé d'immunobuvardage comprenant l'opération consistant à appliquer des solutions anticorps à une membrane au moyen d'un dispositif selon l'une quelconque des revendications 1 à 7.

9. Système comprenant un dispositif selon l'une quelconque des revendications 1 à 7, comprenant en outre une solution activante entre ledit dispositif et ladite membrane.

10. Système selon la revendication 9, dans lequel ladite membrane est constituée de nitrocellulose en membrane ou de polyfluorure de vinylidène (PVDF).

11. Système selon la revendication 9, dans lequel ladite membrane comprend sur elle ou à l'intérieur des peptides, des polypeptides ou des protéines.

12. Système selon la revendication 9, comprenant en outre à l'intérieur des canaux microfluidiques une ou plusieurs solutions contenant des anticorps.

13. Système selon la revendication 12, dans lequel chaque canal microfluidique d'un ensemble de canaux comprend une solution contenant des anticorps différente ou chaque ensemble de canaux comprend la même combinaison de solutions contenant des anticorps.

14. Système selon la revendication 12, dans lequel chaque canal microfluidique comprend une solution contenant des anticorps différente.

15. Procédé d'immunobuvardage comprenant l'opération consistant à appliquer des solutions anticorps à une membrane au moyen d'un système selon l'une quelconque des revendications 9 à 14.
